# EUROPÄISCHE PATENTSCHRIFT

(11) **EP 2 044 441 B1**
(45) Veröffentlichungstag und Bekanntmachung des Hinweises auf die Patenterteilung: **16.01.2013**
(21) Anmeldenummer: 07786166.4
(22) Anmeldetag: 18.07.2007
(51) Int. Cl.: G01N 33/68

(54) **VERFAHREN ZUR STEUERUNG DER THERAPIE VON PATIENTEN MIT HERZINSUFFIZIENZ ANHAND DER IN VITRO BESTIMMUNG VON SCHWELLENWERTEN VON VASOAKTIVEN PEPTIDEN**
METHOD FOR CONTROLLING THE THERAPY OF PATIENTS SUFFERING FROM CARDIAC INSUFFICIENCY BY MEANS OF IN VITRO DETERMINATION OF THRESHOLD VALUES OF VASOACTIVE PEPTIDES
PROCÉDÉ DE COMMANDE DU TRAITEMENT DE PATIENTS SOUFFRANTS D'UNE INSUFFISANCE CARDIAQUE D'APRÈS LA DÉTERMINATION IN VITRO DE VALEURS SEUILS DE PEPTIDES VASOACTIFS

(30) Priorität: 24.07.2006 DE 102006034142
(43) Veröffentlichungstag der Anmeldung: 08.04.2009
(73) Patentinhaber: B.R.A.H.M.S GmbH, 16761 Hennigsdorf (DE)
(72) Erfinder: BERGMANN, Andreas, 13465 Berlin (DE)
(74) Vertreter: Andrae, Steffen
(86) Internationale Anmeldenummer: PCT/EP2007/006393
(87) Internationale Veröffentlichungsnummer: WO 2008/012019

(56) Entgegenhaltungen:
- EP-A- 1 619 505
- KJAER A: "[Neuroendocrine activation in heart insufficiency II. Can diagnosis be confirmed and prognosis evaluated by a blood test?]" UGESKRIFT FOR LAEGER 30 OCT 2000, Bd. 162, Nr. 44, 30. Oktober 2000 (2000-10-30), Seiten 5910-5913, XP002463187 ISSN: 0041-5782
- DE GEVIGNEY ET AL: "Donnees physiopathologiques et implications therapeutiques au cours de l'insuffisance cardiaque gauche" REVUE DE MEDECINE INTERNE, CMR, ASNIERES, FR, Bd. 26, Nr. 11, November 2005 (2005-11), Seiten 874-884, XP005168512 ISSN: 0248-8663
- STRUCK ET AL: "Proteolytic processing pattern of the endothelin-1 precursor in vivo" PEPTIDES, ELSEVIER, AMSTERDAM, US, Bd. 26, Nr. 12, Dezember 2005 (2005-12), Seiten 2482-2486, XP005174851 ISSN: 0196-9781
- STRUCK JOACHIM ET AL: "Identification of an Adrenomedullin precursor fragment in plasma of sepsis patients" PEPTIDES (NEW YORK), Bd. 25, Nr. 8, August 2004 (2004-08), Seiten 1369-1372, XP004551479 ISSN: 0196-9781

## Beschreibung

Die vorliegende Erfindung betrifft eine neue Anwendung eines diagnostischen *in vitro* Verfahrens zur Bestimmung von bestimmten vasoaktiven (auf Gefäße wirkenden; vasotropen) endogenen Peptiden in Blutproben zur Steuerung der Therapie von Patienten mit Herzinsuffizienz, indem der Erfolg oder Misserfolg therapeutischer Interventionen anhand der Veränderung der messbaren Konzentrationen derartiger Peptide beurteilt wird.

Von Herzinsuffizienz (engl. heart failure, HF; cardiac insufficiency) wird gesprochen, wenn das Herz nicht mehr in der Lage ist, den Körper ausreichend mit Blut und damit auch mit Sauerstoff zu versorgen. Die Herzinsuffizienz (Herzleistungsschwäche) ist keine eigenständige Krankheit, sondern ein Krankheitsbild mit verschiedenen Ursachen und mehreren charakteristischen Symptomen. Es werden zur genaueren Charakterisierung der verschiedenen Formen der Herzinsuffizienz je nach Ort der Erkrankung, Art der Symptome oder Art der Krankheitsentwicklung verschiedene Begriffe verwendet, von denen insbesondere zu nennen sind Rechts- bzw. Linksherzinsuffizienz (je nachdem, ob das rechte Herz oder das linke Herz erkrankt ist; left ventricular insufficiency and rightsided heart failure respectively), Globale Herzinsuffizienz (fortgeschrittene Fälle und/oder wenn beide Herzkammern primär betroffen sind; global heart failure), systolische Herzinsuffizienz (der Herzmuskel ist nicht mehr in der Lage, kräftig zu pumpen; systolic heart failure), diastolische Herzinsuffizienz (der Herzmuskel kann nicht mehr richtig entspannen und sich mit Blut füllen; diastolic heart failure), Vorwärts- und Rückwärtsversagen (unzureichende Blutauswurfleistung bzw. Rückstau des Bluts vor dem Herzen; forward heart failure and backward heart failure respectively), Stauungsinsuffizenz (dekompensierte Rechtsherzinsuffizienz; congestive heart failure, CHF), chronische Herzinsuffizienz (die Symptome entwickeln sich langsam über Monate bis Jahre; eine plötzliche akute Verschlechterung ist jederzeit möglich; chronic heart failure) und akute Herzinsuffizienz (Entstehen der Herzinsuffizienz innerhalb von Minuten oder Stunden, z.B. nach einem Herzinfarkt; acute heart failure).

Herzinsuffizienz ist in 70% der Fälle durch eine Pumpschwäche des Myokards aufgrund einer koronaren Herzkrankheit bedingt. Sie betrifft vor allem die ältere Bevölkerung.

Die klassischen diagnostischen Maßnahmen zur Erkennung einer Herzinsuffizienz sind körperliche Untersuchungen von Herz und Lunge und auf Ödeme, EKG (auch als Belastungs- und/oder Langzeit-EKG), Blutdruckmessung, Untraschalluntersuchungen sowie ggf. Röntgenuntersuchungen, Laboruntersuchungen, Lungenfunktionsprüfungen und Bestimmung der Sauerstoffsättigung des Bluts. Je nach klinischem und diagnostischem Befund werden potentielle und erkrankte Herzinsuffizienz-Patienten vier Gruppen zugeordnet, und zwar in Anlehnung an die Empfehlungen der New York Heart Association (NYHA) den vier sogenannten NYHA-Stufen I bis IV.

Für die Zuordnung zu den einzelnen NYHA-Stufen gilt:
NYHA I = keine Beschwerden
NYHA II = Beschwerden bei stärkerer körperlicher Belastung (z.B. Luftnot nach 3 Etagen Treppensteigen oder beim zügigen Bergaufgehen)
NYHA III = Beschwerden bei leichter körperlicher Belastung (z.B. Luftnot nach 1 Etage Treppensteigen, beim langsamen Bergaufgehen oder beim zügigen Gehen in der Ebene)
NYHA IV = Beschwerden bereits in Ruhe, die unter leichter körperlicher Belastung zunehmen.

In jüngerer Zeit wurde zu diagnostischen und prognostischen Zwecken zunehmend auch die Messung von endogenen vasoaktiven Peptiden herangezogen, deren Konzentrationen bei Herzinsuffizienz gegenüber gesunden Kontrollpersonen in charakteristischer Weise verändert sind (sogenannte Kardiovaskulärmarker; vgl. auch (1)). In diesem Zusammenhang sind in erster Linie zu nennen die natriuretischen Peptide (ANP, BNP, CNP) sowie des weiteren die vasoaktiven Peptide Adrenomedullin (ADM), Endothelin-1 (ET-1) und Vasopressin (Arginin-Vasopressin; AVP), um deren Bestimmung es in der vorliegenden Anmeldung geht. Die Freisetzung der letztgenannten drei Peptide wurde erst mit Hilfe von neu entwickelten Assays der Anmelderin, die weiter unten noch genauer erläutert werden, auf einfache und valide Weise messbar. Dabei hat sich gezeigt, dass hohe Konzentrationen von AMP, ET-1 und AVP klare Hinweise auf eine schlechte Prognose bzw. einen wahrscheinlich tödliche Ausgang der Erkrankung liefern.

Zur Basisterapie einer diagnostizierten chronischen Herzinsuffizienz gehört heute vor allem eine Behandlung mit ACE-Hemmern (ACE = angiotensin converting enzyme), Betablockern sowie insbesondere Diuretika (harntreibenden Substanzen). Als weitere bei Herzinsuffizienz eingesetzte Therapeutika sind Angiotensin-II-Rezeptoren-Blocker (ARB), blutverdünnende Mittel, Calciumkanalblocker, Digitalis-Präparate (Digitalisglykoside wie z.B. Digitoxin, Digoxin und davon abgeleitete halbsynthetische Derivate), Vasodilatoren und Maßnahmen wie die Erhöhung der Kaliumzufuhr zu nennen. In schweren Fällen (NYHA IV) werden sog. IV-Diuretika sowie, mit einer geringeren Häufigkeit, Präparate wie Dobutamin, Dopamin, Milrinon, Nesiritid, Nitroglycerin oder Nitroprussid angewandt. Bei schweren chronischen oder akuten Verlaufsformen mit drohenden Komplikationen wie einem Lungenödem oder einem kardiogenen Schock müssen die Patienten aufgrund der kritischen Prognose intensivmedizinisch behandelt werden.

Ein großer Teil der im o.g. Sinne behandelten Patienten reagiert auf die Standardtherapien jedoch nur suboptimal, mit der Konsequenz der Auftretens von "Ereignissen wie Re-Hospitalisierung aufgrund starker Symptome (Atemnot, Kreislaufzusammenbruch) oder Tod. Es ist bisher nur sehr schwer möglich, den Erfolg oder Misserfolg einer gewählten Behandlungsstrategie so frühzeitig zu erkennen, dass die Therapiemaßnahmen gegebenenfalls im Sinne einer Therapieumstellung rechtzeitig geändert werden können, bevor es zu den o.g. "Ereignissen" aufgrund einer Wirkungslosigkeit der vorrangig gewählten Therapiemaßnahmen kommt.

Aus (6) ist es zwar bekannt, dass bei neuroendokrinen Markern, unter denen auch die aktiven Peptide Vasopressin und Endothelin erwährt werden, die Plasma-Level bei Herzversagen erhöht sein können, wobei der Autor allerdings zu dem Schluss kommt, dass Vasopressin keinen prognostischen Wert hat. Zu Endothelin fehlen konkrete Angaben. Der Autor geht nicht auf die Problematik einer reproduzierbaren, zuverlässigen Konzentraticnsbestimmung aktiver Peptide in Serum-, ?lasma- oder Blutproben vcn Patienten ein.

In (7) wird erwähnt, dass die Erwartung besteht, stabile Fragmente des Proendothelins-1 als diagnostische Targets nutzen zu können, auch um therapeutische Maßnahmen zu steuern. Konkrete Angaben zur Behandlung von Herzinsuffizienz-Patienten fehlen jedoch.

BNP wurde und wird für eine Verwendung zur Therapiekontrolle erprobt, jedoch bislang nicht mit einem überzeugenden Erfolg (5) . Es ist daher Aufgabe der vorliegenden Erfindung, dem Arzt neue diagnostische Möglichkeiten zur Verfügung zu stellen, die klare und möglichst frühzeitige Hinweise auf den Erfolg der zur Behandlung einer Herzinsuffizienz gewählten Behandlungsmaßnahmen liefern.

Die genannten "Hinweise" sollten dabei als klare, direkt den relevanten Zustand eines Patienten widerspiegelnde Messergebnisse in Zahlenform erhältlich sein, an denen sich der Arzt direkt orientieren kann, so dass eine "Einstellung des Patienten" im Sinne einer Verbesserung der genannten Messwerte direkt als vordergründiges Therapieziel angestrebt werden kann.

Die genannte Aufgabe wird gemäß der Erfindung durch ein in vitro Verfahren zur Steuerung der therapeutischen Behandlung eines Herzinsuffizienz-Patienten gemäß Anspruch 1 gelöst.

Speziellere bzw. bevorzugte Ausgestaltungen eines solchen Verfahrens beinhalten die in den Ansprüchen 2 bis 5 angegebenen Maßnahmen.

Die im Rahmen des erfindungsgemäßen Verfahrens zu bestimmenden vasoaktiven endogenen Peptide sind solche, deren Ausschüttung sich im Falle von Herzinsuffizienz in charakteristischer Weise verändert. Die vasoaktiven Peptide gehören dabei sowohl zu endogen gebildeten gefäßerweiternden (vasodilatorischen) oder gefäßverengenden (vasokonstriktorischen) physiologisch aktiven Peptiden und sind insbesondere das vasodilatorische Peptid Adrenomedullin (ADM) sowie die vasokonstriktorischen Peptide Endothelin-1 (ET-1) und Vasopressin (AVP). Diese werden vorzugsweise unter Verwendung von Assays der Anmelderin bestimmt, mit denen die Freisetzung der eigentlichen vasoaktiven Peptide anhand der Bestimmung von physiologisch inaktiven Co-Peptiden (MR-proADM, CT-ProET-1, CT-proAVP bzw. Copeptin), die wie die vasoaktiven Peptide aus einem jeweiligen Propeptid-Vorläufer gebildet werden, bestimmt wird.

Ihre Bestimmung im Zusammenhang mit der Therapiesteuerung von Herzinsuffizienz-Patienten kann als auch als Verwendung als kurzfristiger Surrogatmarker für den Erfolg einer Therapie der Herzinsuffizienz bezeichnet werden.

Es ist als Anwendung des erfindungsgemäßen Verfahrens anzusehen, wenn die Bestimmung der o.g. Peptide durch die Bestimmung zusätzlicher klinischer oder biochemischer Parameter ergänzt wird, beispielsweise dadurch, dass man zusätzlich auch noch andere endogene vasoaktive Analyten bestimmt oder mitbestimmt. Es können in diesem Zusammenhang beispielhaft ergänzend genannt werden die Peptide des Renin-Angiotensin-Aldosteron (RAA) Systems, insbesondere das vasokonstriktorische Peptid Angiotensin II, die sog. Substanz P (SP), sowie ferner die vasodilatorischen Peptide CGRP₁₋₃₇, Amylin (IAPP), Endothelin-3 (ET-3) und das vasoaktive Intestinalpeptid (VIP). Ferner ist auch eine Mitberücksichtigung von weiteren Nicht-Peptid-Analyten wie NO (z.B. bestimmbar als Nitrit oder Nitrat) zu nennen.

Wenn von einer Bestimmung der jeweiligen Analyten "im Blut" gesprochen wird, umfasst dieser Begriff die Bestimmung in Vollblut, Serum oder Plasma. Die Bestimmung der Analyten führt man vorzugsweise mit Hilfe immundiagnostischer Bestimmungsverfahren in Form von Immunoassays vom Sandwichtyp durch, z.B. mit einem der nachfolgend erläuterten Assays der Anmelderin.

Die Konzentration von Adrenomedullin (ADM) wird als Konzentration eines midregionalen ProADM-Fragments (MR-proADM) bestimmt, das die Aminosäuren 45-92 des Prä-Proadrenomedullins umfasst. Ein geeigneter Assay ist beschrieben in EP 1 488 209 B1 bzw. WO 2004/090546 bzw. in (2). Unter Verwendung dieses Assays werden bei gesunden Personen ADM-Normalkonzentrationen bestimmt, die im Bereich von 0,2 - 0,6 nmol/l liegen (die Bereichsobergrenze stellt einen Schwellenwert dar, an man sich für die Therapieentscheidung orientiert). Die Einstellung eines Patienten auf eine solche Normalkonzentration stellt das o.g. vordergründige Therapieziel der Behandlung einer Herzinsuffizienz dar.

Die Konzentration von Endothelin 1 (ET-1) wird als Konzentration eines C-terminalen ET-1-Fragments (CT-proET-1) bestimmt, das die Aminosäuren 168-212 des Prä-Proendothelins 1 umfasst. Ein geeigneter Assay ist beschrieben in 1 564 558 B1 bzw. WO 2005/078456 und in (3). Unter Verwendung dieses Assays werden bei gesunden Personen ET-1 Normalkonzentrationen bestimmt, die im Bereich von 25 - 70 pmol/l liegen (die Bereichsobergrenze stellt einen Schwellenwert dar, an man sich für die Therapieentscheidung orientiert). Die Einstellung eines Patienten auf eine solche Normalkonzentration stellt ebenfalls ein o.g. vordergründiges Therapieziel der Behandlung einer Herzinsuffizienz dar.

Für die Bestimmung der Konzentration von Vasopressin (AVP) ist die Verwendung eines Assays empfehlenswert, mit dem das Propeptid-Fragment CT-proAVP (Copeptin) bestimmt wird und der in näheren Einzelheiten beschrieben wird in der Veröffentlichung WO 2006/018315 bzw. in (4). Unter Verwendung dieses Assays werden bei gesunden Personen AVP-Normalkonzentrationen bestimmt, die unter 13 pmol/l (< 13 pmol/l) liegen (der genannte Wert stellt einen Schwellenwert dar, an man sich für die Therapieentscheidung orientiert). Die Einstellung eines Patienten auf eine solche Normalkonzentration stellt ein weiteres vordergründiges Therapieziel der Behandlung einer Herzinsuffizienz dar.

Die Berücksichtigung nur eines der o.g. Parameter, d.h. die Einstellung eines Patienten auf die Normalkonzentration nur eines der o.g. Parameter, liefert, wie nachfolgend anhand von Messergebnissen in einer Tablle 1 gezeigt wird, bereits Aussagen von hoher Signifikanz für den Therapieerfolg bzw. Therapiemisserfolg.

Wie ebenfalls nachfolgend in einer Tabelle 2 gezeigt, wird gemäß der Erfindung ein Behandlungserfolg jedoch mit einer noch deutlich höheren ein Behandlungserfolg jedoch mit einer noch deutlich höheren Signifikanz angezeigt, wenn es gelingt, bei dem Patienten gleichzeitig zwei oder vorzugsweise alle drei genannten Parameter in die angegebenen Normalbereiche zu bringen. Denn obwohl alle Parameter bei Herzinsuffizienz erhöht gefunden werden, sind sie voneinander weitgehend unabhängig und können sich dadurch ergänzen.

Die erfindungsgemäße *in vitro* Bestimmung kann man bei einer klinischen Routineanwendung in größerem Maßstab zweckmäßiger Weise auch als Simultanbestimmung mittels einer Chiptechnologie-Messvorrichtung oder im Rahmen einer sog. Point-of-Care (POC)-Bestimmung mit einer immunchromatographischen Messvorrichtung durchführen. Die Ermittlung und Auswertung des komplexen Messergebnisses einer Multiparameter-Bestimmung erfolgt zweckmäßiger Weise mit Hilfe eines geeigneten Computerprogramms.

Wenn in dieser Anmeldung der Begriff "Konzentration" verwendet wird, meint dieser Begriff nicht, im Sinne einer einengenden Gleichsetzung, die in der biologischen Probe messbaren stationären Konzentration des eigentlichen vasoaktiven Peptids.

Die wichtigsten im Rahmen der vorliegenden Erfindung diskutierten, pathophysiologisch freigesetzten vasoaktiven Peptide liegen nur zu einem geringeren Teil frei bzw. unbehindert messbar in biologischen Flüssigkeiten vor. Wesentliche Teile der pathophysiologisch freigesetzten vasoaktiven Peptide werden der biologischen Flüssigkeit durch Bindung an Rezeptoren und andere Membran- oder Gefäßstrukturen rasch entzogen und/oder abgebaut.

Die Messung von inaktiven, aus den gleichen Vorläufer-Propeptiden gebildeten Co-Peptiden, wie sie gemäß der vorliegenden Erfindung unter Verwendung der hierin genannten Assays der Anmelderin erfolgt, spiegelt, anders als die momentane Konzentration in einer biologischen Flüssigkeit, die Freisetzung der vasoaktiven Peptide im Sinne von "Wirkkonzentrationen" über einen längeren Zeitabschnitt wieder und gestattet eine indirekte Miterfassung auch gebundener oder schnell abgebauter Anteile des vasoaktiven freigesetzten Peptids. Das führt in Verbindung mit der höheren Stabilität solcher Co-Peptide zu höheren messbaren Absolutkonzentrationswerten für den zu bestimmenden Analyten in der biologischen Flüssigkeit, z.B. in Serum oder Plasma.

Die in der vorliegenden Erfindung angesprochenen Konzentrationen sind jedoch nicht notwendigerweise nur die messbaren Konzentrationen solcher inaktiver Co-Peptide, sondern können auch die Konzentrationen anderer messbarer Analyten erfassen, z.B. kleiner Moleküle wie NO, die jeweils in einem im wesentlichen proportionalen Verhältnis zu den für die genannten inaktiven Co-Peptide messbaren Konzentrationen gebildet werden bzw. in der biologischen Flüssigkeit vorhanden sind. Derartige, in einer biologischen Flüssigkeit (Serum, Plasma) in einem proportionalen Verhältnis zu den vasoaktiven Peptiden bzw. Co-Peptiden vorhandene Analyten können als "Surrogate vasoaktiver Peptide" angesehen werden, deren Bestimmung der direkten Bestimmung der vasoaktiven Peptide oder der entsprechenden Co-Peptide gleichwertig sein kann und in gleicher Weise Werte liefern kann, die die Messwerte für die Konzentrationen der vasoaktiven Peptide bzw. der Co-Peptide ersetzen können.

Bei Erkrankungen mit einem eher chronischen Verlauf ohne plötzliche Verschlechterungen oder Verbesserungen des Zustands des Patienten, wie es bei einer chronischen Herzinsuffizienz der Fall sein kann, besteht z.B. eine hohe Wahrscheinlichkeit dafür, dass sich bezüglich der verschiedenen krankheitsrelevanten Analyten ohne therapeutische Intervention oder bei einer regelmäßigen, gleichbleibenden therapeutischen Intervention durch Medikamentenverabreichung ein nur wenig variabler, sich nur langsam verändernder stationärer Gesamtzustand ausbildet. In einem solchen Falle sollten die stationär in der biologischen Flüssigkeit des Patienten messbaren Konzentrationen eines aktiven Analyten, im vorliegenden Falle eines vasoaktiven Peptids, im wesentlichen proportional zu den über längere Zeiträume pathophysiologisch freigesetzten Mengen des gleichen Analyten sein, wie sie in Form von physiologisch inaktiven Co-Peptiden mit den Assays der Anmelderin gemessen werden können. Das bedeutet, dass sich die bei der bevorzugten Multiparameter-Bestimmung inaktiver Co-Peptide gemäß der vorliegenden Erfindung festgestellbaren Abweichungen von den Kontrollwerten Gesunder, sowie der krankheitstypische Gang dieser Abweichungen, auch in den in der Regel niedrigeren stationären Konzentrationen der aktiven Analyten widerspiegeln sollten.

Die Erfindung wird nachfolgend im experimentellen Teil unter Bezugnahme auf zwei Tabellen noch näher erläutert.

Die im experimentellen Teil beschriebenen Messungen von MR-proADM, CT-proAVP und CT-proET-1 in Patientenplasmen erfolgte mit den weiter oben erwähnten, in der dort genannten Literatur beschriebenen Assays der Anmelderin, die alle ihrem Wesen nach nichtkompetitive immunoluminometrische Sandwichassays darstellen.

Nachfolgend wird die Erfindung anhand von Messergebnissen für die Marker ADM, ET-1 und VAP bei Herzinsuffizienz-Patienten noch näher erläutert.

### Experimenteller Teil

### Assaybeschreibung

Die Messung von MR-proADM im Plasma erfolgte mit einem immunoluminometrischen Sandwichassay im wesentlichen so wie im experimentellen Teil der o.g. WO 2004/090546 bzw. in (2) beschrieben wird.

Die Messung von CT-proET-1 im Plasma erfolgte mit einem immunoluminometrischen Sandwichassay im wesentlichen so wie im experimentellen Teil der o.g. WO 2005/078456 bzw. in (3) beschrieben wird.

Die Messung von CT-proAVP (Copeptin) zur Bestimmung von freigesetztem AVP im Plasma erfolgte mit einem immunoluminometrischen Sandwichassay im wesentlichen so wie im experimentellen Teil der WO 2006/018315 bzw. in (4) beschrieben wird.

### Messdatenerhebung:

Zur Prüfung der Frage, ob sich die Bestimmung von ET-1 (als CT-ProET-1), ADM (als MR-proADM) und Vasopressin (als CT-proAVP) allein oder in Kombination zur frühzeitigen Erkennung eines Therapieerfolgs und damit zur Steuerung der Therapie von Herzinsuffizienz-Patienten eignet, wurde bei allen Patienten eines Kollektivs von 377 Patienten, die mit dem Leitsymptom Atemnot in Notfallaufnahmen eingeliefert wurden, unmittelbar nach der Aufnahme eine erste Blutprobe genommen.

Noch am Tag der Notfallaufnahme wurde mit therapeutischen Interventionen begonnen, und 4 Tage nach Beginn der Intervention erfolgte eine zweite Blutabnahme.

Alle Patienten wurden anschließend über einen Zeitraum von 12 Monaten observiert, und das Versterben eines Patienten oder seine Rehospitalisierung aufgrund akuter Herzinsuffizienz wurde als "Ereignis" erfasst.

Das Patientenkollektiv bestand aus insgesamt 377 Personen, 288 männlich und 89 weiblich. Das Durchschnittsalter betrug 67 ± 11 Jahre, wobei das individuelle Alter der Personen zwischen 42 und 91 Jahren lag. Die Zuordnung der Patienten zu den o.g. NYHA-Klassen war wie folgt:
NYHA I = 21 Patienten; NYHA II = 118; NYHA III = 144;
NYHA IV = 94.

Bei insgesamt 354 der 377 Patienten wurde am Tage der Aufnahme für mindestens einen der genannten Marker ein Wert gemessen, der oberhalb des genannten jeweiligen Schwellenwerts bzw. des Normalbereichs für Gesunde lag.

Von diesen 345 Patienten (n = 345) betrug die 12-Monats-Mortalität 17 % (59 Patienten), und die 12-Monats-Rehospitalisierung (einmal oder häufiger) 20 % (70 Patienten). Daraus ergibt sich eine Gesamt-Ereignishäufigkeit von 38 % (129 Patienten).

In der nachfolgenden Tabelle werden die für die einzelnen Marker für Patienten mit Ereignissen (129 Patienten) erhaltenen Messergebnisse am Tag der Notaufnahme sowie am vierten Tag danach, d.h. am Tag 4 nach Beginn der therapeutischen Intervention, wiedergegeben.

**Tabelle 1**

| **Marker/** Messwertbereiche | Patientenzahl n | Patienten mit mindestens 1 Ereignis in 12 Monaten | % Patienten mit Ereignissen |
|---|---|---|---|
| **MR-proADM** | | | |
| > 0,6 nmol/l am Tag der Aufnahme | 340 | 129 | 37,9 |
| > 0,6 nmol/l an Tag 4 | 156 | 125 | 80,1 |
| < 0,6 nmol/l an Tag 4 | 184 | 4 | 2,2 |

| **CT-proET-1** | | | |
|---|---|---|---|
| > 70 pmol/l am Tag der Aufnahme | 331 | 126 | 38,0 |
| > 70 pmol/l an Tag 4 | 141 | 120 | 85,1 |
| < 70 pmol/l an Tag 4 | 190 | 6 | 3,2 |

| **CT-proAVP** | | | |
|---|---|---|---|
| > 13 pmol/l am Tag der Aufnahme | 320 | 121 | 37,8 |
| > 13 pmol/l an Tag 4 | 155 | 110 | 71,0 |
| < 13 pmol/l an Tag 4 | 165 | 11 | 6,7 |

**Tabelle 2**

| Kombinierte Berücksichtigung der Messwerte an Tag 4 von | Patientenzahl n | Patienten mit mindestens 1 Ereignis in 12 Monaten | % Patienten mit Ereignissen |
|---|---|---|---|
| MR-proADM < 0,6 nmol/l plus CT-proET-1 < 70 pmol/l | 172 | 1 | 0,6 |
| zusätzliche Berücksichtigung von CT-proAVP < 13 pmol/l | 148 | 0 | 0 |

Aus Tabelle 1 (letzte Spalte) ist abzulesen, dass bei Berücksichtigung nur eines einzelnen Markers bereits eine signifikante Korrelation zwischen der ereignisfreien 12-Monats-Überlebenszeit und der Normalisierung der Werte für den jeweiligen Marker nach 4 Tagen erhalten wird (nur 2,2 bzw. 3,2 bzw. 6,7 Ereignisse in den Gruppen der "einfach normalisierten Patienten").

Tabelle 2 ist zu entnehmen, dass die Korrelation noch einmal erheblich, im Ergebnis überraschend, verbessert wird, wenn man gleichzeitig die Messergebnisse für MR-proADM sowie für CT-proET-1 berücksichtigt (nur noch 0,6 Ereignisse in der Gruppe der "doppelt normalisierten Patienten"). Wird zusätzlich als dritter Marker CT-proAVP berücksichtgt, wird eine Gruppe "dreifach normalisierter Patienten" erhalten, in der es zu keinerlei Ereignissen mehr kam.

Der Arzt kann somit als kurzfristiges Therapieziel seiner therapeutischen Interventionen die Normalisierung oder zumindest Absenkung der gemessenen Werte für die genannten Marker betrachten. Er könnte auf diese Weise schnell, d.h. bereits nach etwa 4 Tagen (der Zeitraum kann auc etwas kürzer oder auch länger sein), Responder (die auf die gewählte Therapie ansprechen) von Non-Respondern (die auf die gewählte Therapie nicht ansprechen) unterscheiden und bei den Non-Respondern ggf. ein Umstellung der Therapie einleiten. Eine solche frühzeitige Anpassung der Therapie bei einem anhand der messbaren Markerkonzentrationen erkennbaren Ausbleiben des Therapierfolgs kann lebensrettend bzw. lebensverlängernd sein.

Ist der Patient therapieresistent und spricht auf keine der gewählten Therapien an, erleichtert das ggf. die schnelle Entscheidung für schwerwiegendere Eingriffe z.B. chirurgischer Art.

### Literatur

1. ALBERTUS BEISHUIZEN, KOEN J. HARTEMINK, ISTVAN VERMES, AB JOHAN GROENEVELD (2005), Circulating cardiovascular markers and mediators in acute illness: an update. Clinica Chimica Acta 354 (2005) 21-34
2. NILS G. MORGENTHALER, JOACHIM STRUCK, CHRISTINE ALONSO, ANDREAS BERGMANN, Measurement of Midregional Proadrenomedullin in Plasma with an Immunoluminometric Assay, Clinical Chemistry 51:10, 2005, 1823-1829
3. JANA PAPASSOTIRIOU, NILS G. MORGENTHALER, JOACHIM STRUCK, CHRISTINE ALONSO, ANDREAS BERGMANN, Immunoluminometric Assay for Measurement of the C-Terminal Endothelin-1 Precursor Fragment in Human Plasma, Clinical Chemistry 52:6, 2006, 1144-1151
4. NILS G. MORGENTHALER, JOACHIM STRUCK, CHRISTINE ALONSO, ANDREAS BERGMANN, Assay for the Measurement of Copeptin, a Stable Peptide Derived from the Precursor of Vasopressin, Clinical Chemistry 52:1, 2006, 112-119
5. VAN CHENG, RADMILA KAZANAGAR, ALEX GARCIA, LESLIE LE-NERT, PADMA KRISHNASWAMY, NANCY GARDETTO, PAUL CLOPTON, ALAN MAISEL, A Rapid Bedside Test for B-Type Peptidre Prdicts Treatment Outcomes in Patients Admitted for Decompensated Herat Failure: A Pilot Study, J Am Coll Cardiol 2001; 37:386-391
6. KJAER A: ["Neuroendocrine activation in heart insufficiency II. Can diagnosis be confirmed and prognosis evaluated by a blood test?"] UGESKRIFT FOR LAEGER 30 OCT 2000, Bd. 162, Nr. 44, 30. Oktober 2000, Seiten 5910-5913,
7. STRUCK ET AL: "Proteolytic processing pattern of the endothelin-1 precursor in vivo", PEPTIDES, ELSEVIER, AMSTERDAM, US, Bd. 26, Nr. 12, Dezember 2005, Seiten 2482-2486,

## Patentansprüche

1. Verfahren zur Steuerung der therapeutischen Behandlung eines Herzinsuffizienz-Patienten, bei dem man nach Beginn einer therapeutischen Behandlung in vitro in einer Vollblut-, Serum- oder Plasmaprobe des Patienten mittels eines Immunoassays die Änderung der vor Therapiebeginn gemessenen Konzentration von mindestens zwei der vasoaktiven Peptide Adrenomedullin (ADM), Endothelin-1 (ET-1) und/oder Vasopressin (AVP), gemessen als Freisetzung der genannten vasoaktiven Peptide, verfolgt, wobei man zur Messung einen Assay einsetzt, der die Konzentration eines inaktiven Co-Peptids bestimmt, das aus dem gleichen Propeptid-Vorläufer wie das zu bestimmende vasoaktive Peptid gebildet wird, und wobei man im Falle einer ausbleibenden oder unzureichenden Verminderung der genannten Konzentration die Therapiemaßnahmen als unbefriedigend beurteilt und die Therapiemaßnahmen ändert, während man bei einer ausreichenden Verminderung der genannten Konzentration die Therapie als erfolgreich beurteilt, wobei man
(i) die Konzentration von ADM mit Hilfe eines Sandwich-Immunoassays bestimmt, der die Bestimmung der Konzentration von MR-proADM ermöglicht, und eine unzureichende Verminderung der Konzentrationen dann vorliegt, wenn die bei der weiteren Messung ermittelte Konzentration von MR-proADM oberhalb eines Werts von 0,6 nmol/l liegt,
(ii) die Konzentration von ET-1 mit Hilfe eines Sandwich-Immnoassays bestimmt, der die Bestimmung der Konzentration von CT-proET-1 ermöglicht, und eine unzureichende Verminderung der Konzentrationen dann vorliegt, wenn die bei der weiteren Messung ermittelte Konzentration von CT-proET-1 oberhalb eines Werts von 70 pmol/l liegt, und
(iii) die Konzentration von AVP mit Hilfe eines Sandwich-Immnoassays bestimmt, der die Bestimmung der Konzentration von CT-proAVP ermöglicht, und eine unzureichende Verminderung der Konzentrationen dann vorliegt, wenn die bei der weiteren Messung ermittelte Konzentration von CT-proAVP oberhalb eines Werts von 13 pmol/l liegt.

2. Verfahren nach Anspruch 1, **dadurch gekennzeichnet, dass** man die Konzentration von mindestens zwei der genannten vasoaktiven Peptide im Blut des Patienten direkt vor Therapiebeginn bestimmt und dann innerhalb eines Zeitraum von 3 bis 10 Tagen, vorzugsweise nach 4 Tagen, eine weitere Messung der Konzentration der vor Therapiebeginn bestimmten vasoaktiven Peptide vornimmt.

3. Verfahren nach Anspruch 1 oder 2, **dadurch gekennzeichnet, dass** die Verminderung als ausreichend und die Therapie als erfolgreich angesehen wird, wenn der bei der weiteren Messung für zwei der vasoaktiven Peptide oder für alle drei vasoaktiven Peptide bestimmte Konzentrationswert unterhalb der in Anspruch 1 angegebenen Werte für das jeweilige vasoaktive Peptid liegt.

4. Verfahren nach einem der Ansprüche 1 bis 3, **dadurch gekennzeichnet, dass** die Multiparameter-Bestimmung als Simultanbestimmung mittels einer Chiptechnologie-Messvorrichtung erfolgt oder mittels einer immunchromatographischen Messvorrichtung erfolgt, die eine Unterschreitung der Werte oder Obergrenzen für die Normalbereiche für das jeweilige vasoaktive Peptid deutlich anzeigt.

5. Verfahren nach Anspruch einem der Ansprüche 1 bis 4, **dadurch gekennzeichnet, dass** die Ermittlung und Auswertung des komplexen Messergebnisses einer Multiparameter-Bestimmung mit Hilfe eines Computerprogramms erfolgt.

## Claims

1. A method for controlling the therapeutic treatment of a patient suffering from cardiac insufficiency, in which, after the beginning of a therapy, in *vitro* in a whole blood, serum or plasma sample of the patient by means of an immunoassay the change of the concentration, which has been measured before the beginning of the treatment, of at least two of the vasoactive peptides adrenomedullin (ADM), endothelin-1 (ET-1) and/or vasopressin (AVP), measured as release of said vasoactive peptides, is monitored with the use of an assay which determines the concentration of an inactive copeptide formed from the same propeptide precursor as the vasoactive peptide to be determined, and in the case of absence of a reduction or an insufficient reduction of said concentration, the therapeutic measures are assessed as unsatisfactory and the therapeutic measures are changed, whereas, in the case of a sufficient reduction in said concentration, a successful therapy is assumed, wherein
(i) the concentration of ADM is determined with the aid of a sandwich immunoassay which permits the determination of the concentration of MR-proADM and an insufficient reduction in the concentrations is present when the concentration of MR-proADM, determined in the further measurement, is above a value of 0.6 nmol/l,
(ii) the concentration of ET-1 is determined with the aid of a sandwich immunoassay which permits the determination of the concentration of CT-proET-1 and an insufficient reduction in the concentrations is present when the concentration of CT-proET-1, determined in the further measurement, is above a value of 70 pmol/l, and
(iii) the concentration of AVP is determined with the aid of a sandwich immunoassay which permits the determination of the concentration of CT-proAVP and an insufficient reduction in the concentrations is present when the concentration of CT-proAVP, determined in the further measurement, is above a value of 13 pmol/l.

2. The method according to Claim 1, **characterized in that** the concentration of at least two of said vasoactive peptides in the blood of the patient is determined directly before the beginning of therapy and a further measurement of the vasoactive peptides determined before the beginning of therapy is carried out within a period of 3 to 10 days, preferably after 4 days.

3. The method according to Claim 1 or 2, **characterized in that** the reduction is regarded as sufficient and the therapy as successful if the concentration value determined in the further measurement for two of the vasoactive peptides or for all three vasoactive peptides is below the values stated in Claim 1 for the respective vasoactive peptide.

4. The method according to any of Claims 1 to 3, **characterized in that** the multiparameter determination is effected as a simultaneous determination by means of a chip technology measuring apparatus or is effected by means of an immunochromatographic measuring device which clearly indicates that the values or the upper limits for the normal ranges for the respective vasoactive peptide have not been reached.

5. The method according to any of Claims 1 to 4, **characterized in that** the determination and evaluation of the complex measured result of a multiparameter determination is effected with the aid of a computer program.

## Revendications

1. Procédé de contrôle du traitement thérapeutique d'un patient souffrant d'insuffisance cardiaque, dans lequel, après le début d'une thérapie, in vitro dans un échantillon de sang total, de sérum ou de plasma du patient au moyen d'un dosage immunologique le changement de la concentration, qui a été mesurée avant le début du traitement, d'au moins deux des peptides vasoactifs adrénomédulline (ADM), endothéline-1 (ET-1) et ou vasopressine (AVP), mesurés en tant que libération desdits peptides vasoactifs, est contrôlé à l'aide d'un dosage qui détermine la concentration d'un copeptide inactif formé à partir du même précurseur propeptidique que le peptide vasoactif à déterminer, et dans le cas d'une absence de réduction ou d'une réduction insuffisante de ladite concentration, les mesures thérapeutiques sont évaluées comme étant insatisfaisantes et les mesures thérapeutiques sont changées, tandis que, dans le cas d'une réduction suffisante de ladite concentration, une thérapie réussie est présumée, où
(i) la concentration en ADM est déterminée à l'aide d'un dosage immunologique sandwich qui permet la détermination de la concentration en MR-proADM et une réduction insuffisante des concentrations est présente lorsque la concentration en MR-proADM, déterminée dans la suite de la mesure, est supérieure à une valeur de 0,6 nmol/l,
(ii) la concentration en ET-1 est déterminée à l'aide d'un dosage immunologique sandwich qui permet la détermination de la concentration en CT-proET-1 et une réduction insuffisante des concentrations est présente lorsque la concentration en CT-proET-1, déterminée dans la suite de la mesure, est supérieure à une valeur de 70 pmol/l, et
(iii) la concentration en AVP est déterminée à l'aide d'un dosage immunologique sandwich qui permet la détermination de la concentration en CT-proAVP et une réduction insuffisante des concentrations est présente lorsque la concentration en CT-proAVP, déterminée dans la suite de la mesure, est supérieure à une valeur de 13 pmol/l.

2. Procédé selon la revendication 1, **caractérisé en ce que** la concentration d'au moins deux desdits peptides vasoactifs dans le sang du patient est déterminée directement avant le début de la thérapie et la suite de la mesure des peptides vasoactifs déterminée avant le début de la thérapie est réalisée au cours d'une période de 3 à 10 jours, de préférence au bout de 4 jours.

3. Procédé selon la revendication 1 ou 2, **caractérisé en ce que** la réduction est considérée comme étant suffisante et la thérapie comme étant réussie si la valeur de concentration déterminée dans la suite de la mesure pour deux des peptides vasoactifs ou pour l'ensemble des trois peptides vasoactifs est inférieure aux valeurs indiquées dans la revendication 1 pour le peptide vasoactif respectif.

4. Procédé selon l'une quelconque des revendications 1 à 3, **caractérisé en ce que** la détermination à paramètres multiples est effectuée en tant que détermination simultanée au moyen d'un appareil de mesure utilisant une technologie à puces ou est effectuée au moyen d'un dispositif de mesure immunochromatographique qui indique clairement que les valeurs ou les limites supérieures des plages normales pour le peptide vasoactif respectif n'ont pas été atteintes.

5. Procédé selon l'une quelconque des revendications 1 à 4, **caractérisé en ce que** la détermination et l'évaluation du résultat mesuré complexe d'une détermination à paramètres multiples sont effectuées à l'aide d'un programme informatique.
